# EUROPÄISCHE PATENTANMELDUNG

(11) **EP 2 415 388 A1**
(43) Veröffentlichungstag der Anmeldung: **08.02.2012**
(21) Anmeldenummer: 11176126.8
(22) Anmeldetag: 01.08.2011
(51) Int. Cl.: A61B 1/00, A61B 1/07, G02B 23/24

(54) **Endoskop mit einstellbarer Blickrichtung**

(30) Priorität: 04.08.2010 DE 102010033427
(71) Anmelder: Karl Storz GmbH & Co. KG, 78532 Tuttlingen (DE)
(72) Erfinder: Fang, Lei, Dr., 78532 Tuttlingen (DE); Weiger, Ulrich, 78532 Tuttlingen (DE)
(74) Vertreter: Straub, Bernd

(57) **Zusammenfassung**

Ein Endoskop (10) mit einstellbarer Blickrichtung (21, 22) umfasst eine einer Mehrzahl von Lichtaustrittseinrichtungen (32 , 42, 52) am distalen Ende (11) des Endoskops (10). wobei die Lichtausrittseinrichtungen (32, 42, 52) ausgebildet und angeordnet sind, um aus verschiedenen Lichtaustrittseinrichtungen (32, 42, 52) austretendes Beleuchtungslicht in zumindest teilweise unterschiedliche Raumwinkelberiche (34, 44, 54) zu leiten, mehrere Lichtwellenleiter (30, 40, 50) zum Leiten von Beleuchtungslicht zu den Lichtaustrittseinrichtungen (32, 42, 52) und eine Lichtweicheneinrichtung (60; 70; 80) zum steuerbaren Einkoppeln von Beleuchtungslicht in eine oder mehrere der Mehrzahl von Lichtwellenleiter (36).

## Beschreibung

Die vorliegende Erfindung bezieht sich auf ein Endoskop mit einstellbarer Blickrichtung und ein Verfahren zum Beleuchten eines einstellbaren Sichtfelds.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung einen Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im unglinstigsten Fall mass beispielsweise während eines medizinischen Eingriffs mehrfach das Endoskop gewechselt werden. In solchen Fällen ist die Verwendung eines Endoskops mit einer in situ einstellbaren bzw, verstellbaren Blickrichtung vorteilhaft.

Die Benbachtung eines Gegenstands in einem Hohlraum mittels eines Endoskops setzt in der Regel eine Beleuchtung des Gegenstands voraus. Dazu weist ein Endoskop beispielsweise Lichtwellenleiter, insbesondere Glasfasem, auf, mittels derer Beleuchtungslicht vom proximalen Ende des Endoskops entlang des Schafts zum distalen Ende des Endoskops übertragen wird. Lichtaustrittsflächen der Lichtwellenleiter am distalen Ende des Endoskops sind so angeordnet und ausgebildet, dass das Gesamtsichtfeld bzw. Blickfeld ausreichend ausgeleuchtet wird.

Bei einem Endoskop mit einstellbarer Blickrichtung wird das Beleuchtungslicht am distalen Ende des Endoskops im einfachsten Fall so verteilt, dass unabhängig von der jeweils eingestellten Blickrichtung das gesamte Sichtfeld ausgeleuchtet ist. Dies hat jedoch eine Reihe von Nachteilen zur Folge. Insbesondere wird Lichtleistung verschwendet, weil ständig unabhängig von der tatsächlich eingestellten Blickrichtung die gesamten Sichtfelder aller einstellbaren Blickrichtungen ausgeleuchtet werden. Bei einer vorbestimmten erwünschten Helligkeit muss somit insgesamt eine deutlich höhere Lichtleistung zur Verfügung gestellt werden als bei einem Endoskop mit feststehender Blickrichtung.

Ein weiterer Nachteil rührt daher, dass Beleuchtungslicht hoher Intensität Gewebe oder andere Gegenstände photothermisch oder photochemisch schädigen kann. Bei einem Endoskop mit feststehender Blickrichtung fällt ein zu geringer Abstand des distalen Endes des Endoskops zu einem Gegenstand zumindest bei Betrachtung des erfassten Bilds in der Regel auf, Bei Verwendung einer Kamera am Endoskop ist auch eine automatische Warnung von Benutzern möglich, wenn die Helligkeit eines erfassten Bilds eine vorbestimmte Schwelle überschreitet. Bei einem Endoskop mit einstellbarer Blickrichtung fällt jedoch ein Teil des Beleuchtungslichts auf Gegenstände, die auβerhalb des Sichtfelds liegen. Eine unerwünschte Annäberung des distalen Endes des Endoskops an diese Gegenstände und eine resultierende Bestrahlung dieser Gegenstände mit einer zu hohen Strahlungsleistung fällt deshalb nicht auf.

Ein weiterer Nachteil besteht darin, dass auch Beleuchtungslicht, das zunächst außerhalb des Sichtfelds abgestrahlt wird, von Gegenständen oder opaken Medien gestreut oder reflektiert werden kann. Das reflektierte oder gestreute Beleuchtungslisht kann direkt oder indirekt in den Beobachtungsstrahlengung gelangen. Dadurch können Kontraste und von allem in dunklen Bildebereichen die Unterscheidbarkeit von Gegenständen verringert werden.

Ein weiterer Nachteil rührt daher, dass die Beleuchtungsstärke bzw, die Intensität des Beleuchtungslichts in der Richtung, in der die Blickrichtung variiert werden kann (oft auch als vertikale Richtung bezeichnet) im Wesentlichen Konstant ist, während sie in der dazu senkrechten Richtung (oft auch als horizontale Richtung bezeichnet) in der Regel zum Rand des Sichtfelds hin leicht abnimmt Von Endoskopen mit feststehender Blickrichtung sind Benutzer jedoch in der Regel eine Beleuchtungsstärke gewohnt, die sawohl in horizontaler als auch in vertikaler Richtung zum Rand des Sichtfelds hin leicht abnimmt. Die in vertikaler Richtung konstante Beleuchtungsstärke kann deshalb als irritierend empfunden werden.

In der DE 600 15 375 T2 ist eine Anordung mehrerer Prismen beschrieben. Eines der Prismen ist um eine Achse rotierbar, um Beleuchtungslicht in eine einstellbare Blickrichtung zu werfen. Die Prismen nehmen jedoch Raum ein, der vor allem bei immer kleineren Schaftquerschuitten nicht mehr zur Verfügung steht.

Eine Aufgabe der vorliegenden Erfindung besteht darin, ein hinsichtlich der Beleuchtung verbessertes Endoskop mit einstellbarer Blickrichtung und ein verbessertes Verfahren zum Beleuchten eines einstellbaren Blickfelds zu schaffen,

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, Beleuchtungslicht für mehrere verschiedene Blickrichtungen mittels einer Licht eicheneinrichtung in mehrere verschiedene Lichtwellenleiter einzukoppeln, über die das Beleuchtungslicht zu mehreren vershiedenen Lichtaustrittseinrichtungen übertragen wird, die das Beleuchtungslicht in unterschiedliche Raumwinkelbereiche abstrahlen. Die Lichtweicheneinrichtung kann am proximalen Ende des Endoskops augeordnet sein, wo in der Regel deutlich mehr Bauraum zur Verfügung steht, als am distalen Ende des Endoskops.

Ein Endoskops mit einstellbarer Blickrichtung umfasst eine Mehrzahl von Lichtaustrittseinrictungen am distalen Ende des Endoskops_{;} wobei die Lichtaustrittseinrichtungen ausgebildet und angeordnet sind, um aus verschiedenen Lichtaustrittseinrichtungen austretendes Beleuchtungslicht in zumindest teilweise unterschiedliche Raumwinkelbereiche zu leiten; mehrere Lichtwellenleiter zum Leiten von Beleuchtungslicht zu den Lichtaustrittseinrichtungen; und eine Lichtweicheneinrichtung zum steuerbaren Einkoppeln von Beleuchtungslicht in einen oder mehrere der Mehrzahl von Lichtwellenleiter.

Die Blickrichtung des Endoskops ist insbesondere um eine Schwenkachse schwenkbar, die senkrecht zur Längsachse des Endoskops ist. Für die Schwenkbarkeit der Blickrichtung ist beispielsweise ein schwenkbares Prisma oder ein schwenkbarer Spiegel im Beobachtungs-Strahlengang des Endoskops vorgesehen.

Jeder Lichtwellenleiter umfasst beispielsweise eine Glas- oder Kunstrstofffaser oder ein Bündel von Glas- oder Kunststofffasem. Die Faser oder die Fasern können Singlemode- oder Multimode-Fasem sein. Alternativ umfasst jeder Lichtwellenleiter eine oder mehrere Flüssig-Lichtleiter. Die Lichteintrittsflächen der Lichtwellenleiter sind insbesondere am proximalen. Ende des Endoskops angeordnet. Jedem Lichtwellenleiter ist insbesondere genau eine Lichtaustrittseinrichtung zugeordnet

Die Lichtaustrittseinrichtungen umfassen insbesondere Lichtaustrittsflächen an distalen Enden der Lichtwellenleiter. Wenn die Lichtwellenleiter Glas- oder Kunststofffasern umfassen, können deren distale Enden in Öffnungen am distalen Ende des Endoskops gefügt sein, beispielsweise mittels eines Kitts. Die Lichtaustrittseinrichtungen werden dabei durch Lichtaustrittsflächen gebildet, die nach dem Aushärten des Kitts durch Abschleifen und Polieren der ursprünglich überstehenden Enden der Fasern entstehen.

Alternativ weisen die Lichtaustrittseinrichteungen beispielsweise Linsen und oder Abdeckgläser auf. Unabhängig von der Ausgestaltung der Lichtaustrittseinrichtungen kann jeder Lichtaustrittseinrichtung genau ein Lichtwellenleiter oder eine Gruppe von Lichtwellenleitem zugeordnet sein, beispielsweise ein Bündel von Glasfasern.

Das hier beschriebene Endoskop mit mehreren Lichtaustrittseinrichtungen, Lichtwellenleitem zum Leiten von Beleuchtungslicht zu den Lichtaustrittseinrichtungen und einer Lichtweicheneinrichtung zum steuerbaren Einkoppeln von Beleuchtungslicht in die Lichtwellenleiter ermöglicht es, nur das Sichtfeld in der momentan eingestellten Blickrichtung zu beleuchten oder auszuleuchten. Die Strahlungsleistung, die in Raumwikelbereiche gelenkt wird, die auβerhalb des mementanen Sichtfelds liegt, wird dadurch zumindest stark reduziert. Dies reduziert die erforderliche Gesamistrahlungsleistung, das Risiko von photothermischen oder photoschemischen Schäden und die Intensität von Streulicht, das den Bildkontrast reduziert. Ferner kann bei entsprechender ausgestaltung der Lichtaustrittseinrichtungen und der Lichtweicheneinrichtung eine von Verwendern als angenehm empfundene Verteilung des Beleuchtungslichts erzielt werden, bei der die Beleuchtungsstärke zu allen Rändem des Sichtfelds hin leicht abnimmt

Die Verwendung von Lichtwellenleitern zwischen der Lichtweicheneinrichtung und den Lichtaustrittseinrichtungen ermöglicht einen vergröβerten Freiraum bei der Konstruktion. Insbesondere kann die Lichtwecheneinrichtung weit beabstandet vom distalen Ende des Endoskops angeordnet sein, beispielsweise am proximalen Ende des Endoskops, wo in der Regel mehr Bauraum zur Verfügung steht. Die Lichtwellenleiter und die Lichtaustrittseinrichtungen nehmen nur sehr wenig Raum ein und können deshalb selbst in Endoskopen mit sehr geringen Querschnitten verlegt werden. Dies gilt insbesondere bei. Lichtaustrittseinrichtungen in Form einfacher distaler Lichtaustrittsflächen der Lichtwellenleiter.

Mittels einer einfachen mechanischen Einrichtung oder auch mittels einer elektrischen oder magnetischen Einrichtung kann die Lichtweicheneinrichtung so gesteuert werden, dass simultan mit einer Veränderung der Blickrichtung des Endoskops das Beleuchtungslicht jeweils in andere Lichtwellenleiter eingekoppelt wird, um den ausgeleuchteten Raumwinkelbereich jeweils an das momentane Sichtfeld anzupassen, Handhabungseinrichtungen zum Steuern bzw. Einstellen der Blickrichtung sind typischerweise am proximalen Ende des Endoskops angeordnet. Jede an einer Handhabungseinrichtung vorgenommene Einstellung kann deshalb unmittelbar an eine ebenfalls am proximalen Ende des Endoskops angeordnete Lichtweicheneinrichtung übertragen werden.

Bei einem Endoskop, wie es hier beschrieben ist, kann die Lichtweicheneinrichtung ein in mehrere PositionenbewegbaresPrisma umfassen, wobei bei verschiedenen Positionen des Prismas Belenchtungslicht in verschledene Lichtwellenleiter einkoppelbar ist.

Das Prisma ist insbesondere kontinuierlich bewegbar bzw. kann in Positionenbewegt werden bzw. Positioneneinnehmen, die ein Kontinuum bilden. Alternativ sind beispielsweise mittels mechanischer Rasteinrichtungen diskrete Posítíonen vorbestimmt, die das Prisma eínnehmen kann.

Das Prisma íst insbesondere um eine Achse schwenkbar bzw. rotierbar, wobei das bereitgestellte Beleuchtungslicht im Wesentlichen parallel zu dieser Achse auf das Prisma fällt. Durch eine Rotation des Prismas kann die Richtung des vom Prisma abgelenkten Beleuchtungslicht insbesondere auf einemKegelmantelvaniert werden. Im einfachsten Fall liegen die Lichteintrittsflüchen bzw, die proximalen Enden der Lichiwellenleiter auf diesem Kegelmantel.

Bei einem Endoskop,wie es hier beschrieben ist, kann die Lichtweicheneinrichtung ein rotierbares Paar miteinander starr verbundener Prismen umfassen.

Das Paar miteinander starr verhundener Prismen ist insbesondere mittels eines transparenten Kitts verbunden. Die Brechungsindizesder Materialien der beiden Prismen können eine gegenläufige Wellenlängenabhängigkeit aufweisen, um eine Wellenlängenabhängigkeit der Ablenkung des Beleuchtungslichts durch das Prinsmenpaar zu reduzieren oder zu unterdrücken.

Die Lichteintrittstläche und die Lichtaustrittsfläche des Paares miteinander starr verbundener Prismen sind insbesondere parallel zueinander. Dies kann eine präzise Führung des rotierbaren Prismenpaares vereinfachen.Insbesondere im Fall paralleler Lichteintritts-und -austrittsflächen des Prismenpaares weisen die beiden PrismenMaterialienmit unterschiedlichen Brechungsindizes auf.

Bei einem Endoskop, wie es hier kann die Lichtweicheneinrichtung zwei relativ zueinander rotierbare Paare von Prismen umfassen.

Die Prismen eines Paares weisen unterschiedliche Brechungsindlizes auf und können miteinander starr verbunden, insbesondere verkittet sein. Im fall einer unabhlingigen Rotierbarkeit der zwei Prismenpaare ist ein auf die Lichtweicheneinrichtung fallender Lichtstrahl in zweiDimensioneninnerhalbvorbestimmter Grenzen beliebig ablenkbar. Beleuchtungslicht kann somit beispielsweise auf Lichteintrittsflâchen von Lichtwellenleitern gerichtet werden, die in einem Array angeordnet sind,

Wenn beide Prismenpaare gegenläufig zueinander rotieren, kann Beleuchtungslicht, das auf die Lichtweicheneinrichtung fällt, beispielsweise auf Lichteintrittsflächen gerichtet werden, die entlang einer geraden oder einer anderen Kurve angeordnet sind, um das Beleuchtungslicht in die Lichteintrittsflächen einzukoppeln. Zur gegenläufigen Rotation der Prismenpaare sind diese beispielsweise über ein entsprechendes Getriebe miteinander mechanisch gekoppelt.

Die beschriebenen Lichtweicheneinrichtungen mit einem oder mehreren einzeln oder paarweise starr miteinander verbundenen Prismen bilden eine einfache, robuste und gagenüber einer Fehljustage unempfindliche Lichtweicheneinrichtung,die innerhalb eines kleinen Bauraums realisierbar ist.

Bei einem Endoskop,wie es hierbeschrieben ist, kann die Lichtweicheneinrichtungzwei nicht mischbare Flüssigkeiten mit unterschiedlichen Brechungsindizes und eine Grenzflächenstenereinrichtung zum elektrischen oder magnetischen Verändern zumindest entweder der Anordnung oder der Form einer Grenzfläche zwischen den nicht mischbaren Flüssigkeiten umfassen.

Die beschriebene Anordnung zweier nicht mischbarer Flüssigkeiten mit unterschiedlichen Brechangsindizes wird auch als Fllüssiglinse bezeichnet.Durch Veränderung der Krümmung der Grenzfläche zwischens den nicht mischbaren Flüssigkeiten mit unterschiedlichen Brechungsindizes kann die Brechkraft bzw.dieBrennweiteder Flüssiglinse verändert werden Durch ein Kippen einer im Wesentlichen ebenen Grenzflächezwischen nicht mischbaren Flüssigkeiten mit unterschiedlichen Brechungsindizes wirken die beiden Flüssigkeiten wie ein Prisma mit variabler Ablenkung Damit kann bereitgestelltes Beleuchtungslicht auf eine oder mehrere Lichteintriusflächen von Lichtwellenteitem gelenkt werden um das Beleuchtangslicht in den oder die. Lichtwellenleiter einzukoppeln,Durch eine variation der Krümmung der Grenzfläche zwischen den nicht mischbaren Flüssigkeiten kann die Anzahl der Lichteintriusflächen.auf die das Beleuchiungslicht gleichzeitig gelenkt wird, verändert werden. Die beschriebene Lichtweicheneinrichtung mit zwei nicht mischbarenFlüssigkeiten ermöglicht auf diese Weise eine sehr schnelle und flexible Steuerung der Einkopplung von Beleuchtungslicht in einen oder mehrere der Mehrzahl von Lichtwellenleitern.

Bei einem Endoskop, bei dem die Lichtweicheneinrichtung zwei nicht mischbare Flüssigkeiten und eine Grenzflächensteuereinrichtung um fasst,kann die Grenzflächensteuereinrichtung mehrere Elektroden umfassen, wobei die Flüssigkeiten so ausgebildet und die Elektroden so ausgebildet und angeordnet sind,dassdurch Anlegen einer elektrostatisehen Spannung an die Elektroden die Grenzflächezwischen den nicht mischbaren Flussigkeiten zumindest entweder verschoben oder verformt werden kann

Durch die elektrostatischeVerschiebung und/oder Verformung der Grenzfläche zwishen den nicht mischbaren Flüssigkeiten mit unterschiedlichen Brechungsindizes kann Beleuchtungslicht in verschiedene Lichtwellenleiter eingekoppelt werden voraussetzung ist dass zumindest eine der beiden nicht mischbarenFlüssigkeiten durch elektrostatische Felder beeinflusst,insbesondereangezogen oder abgestoßenwerden kann, Alternativ ist zumindest eine der beiden nicht mischbaren Flüssigkeiten durch ein magnetisches Feld beeinflussbar,und die Grenzflächensteuereinrichtungumfasst einen oder mehrere Elektomagneten oder ein oder mehrere bewegbare Permanentmagneten,

Bei einem Endoskop wie es hier beschrieben ist, kann die Lichtweicheneinrichtung eine in mehrere Positionen bewegbare reflektierende Fläche zum Umlenken von Beleuchtungslicht umfassen, wobei bei verschiedenen Positionen der reflektierenden Fläche Beleuchtungslicht in verschiedene Lichtwellenleiter einkoppelbar ist.

Die reflektierende Fläche ist insbesondere kontinuierlich bewegbar bzw. in jede Position aus einem Kontinuum von Positionen bewegbar. Die reflektierende Fläche ist insbesondere translatorischoder rotatorisch bewegbar. Die reflektierende Fläche ist beispielsweise eine verspiegelte oder totalreflektierende Flüche eines spiegels oder eines Prismas bzw. eines transparenten Körpers. Das Verschieben oder Rotieren bzw. Schwenken einer reflektierenden Fläche ermöglicht auf einfache Weise ein Lenken des Beleuchtungslichtsauf eine oder mehrere ausgewählte Lichteintrittsflächen von Lichiwellenleitem.

Bei einem Endoskop wie es hier beschrieben ist sind die Lichteintrittsflächen der LichtWellenleiter und die Lichtweicheneinrichtung insbesondere am proximalen Ende des Endeskops angeordnet.

Wie, bereits erwähnt, ermöglicht die Verwendung der Lichtwellenleiter eine große gestalte rische Freiheit und insbesondere eine nahezu beliebiger Anordnung der Lichtweicheneinrichtung. Die Anordnung der Lichtweicheneinrichtung am proximalen Ende des Endoskops ermöglicht eine Ausbildung des Schafts des Endoskops mit sehr geringem Querschnitt ohne die Lichtweicheneinrichtung besonders Klein ausführen zu müssen.

Wie bereits erwähnt, hat die Anordnung der Lichtweicheneinrichtung am proximalen Ende des Endoskops ferner den Vorteil,dass der Abstand zwischen der Lichtweicheneinrichtung und ebenfalls am proximalen Ende des Endoskops angeordneten Handhabungseinrichtungen klein sein kann. Die Lichtweicheneinrichtung kann somit mit geringem konstruktivem Aufwand unmittelbar und präzise gesteuert werden.

Bei einem Verfahren zum Beleuchten eines Sichtfelds in einer einstellbaren Blickrichtung eines Endoskops wird Beleuchtungslicht bereitgestellt und das bereitgestellte Beleuchtungslich in einen oder mehrere einer momentan eingestellen Blickrichtung zugeordnete Lichtwellenleiter aus einer Mehrzahl von Lichtwellenleitern eingekoppelt. Das eingekoppelte Beleuchtungslicht wird mittels des oder der ausgewählten Lichtwellenleiter zu einer oder mehreren zugeordneten Lichtaustrittseinrichtungen am distalen Ende des Endoskops übertragen Das mittels eines Lichtwellenleiters übertragene Beleuchtungslicht wird mittels einer dem Lichtwellenleiter zugeordneten Lichtaustrittseinrichtung in einen dem Sichtfeld entsprechenden Raumwinkelbereich gelenkt,

Ein verfahren,wie es hier beschrieben ist ist insbesondere mit einem Endoskop durchführbar wie es hier beschrieben ist.

Bei einemVerfahren,wie es hier beschrieben ist, können ferner eine ausgewählte Blickrichtung des Endoskops eingestellt und eine Lichtweicheneinrichtung entsprechend der eingestellten Blickrichtung des Endoskops eingestellt werden

### Kurzbeschreibung der Figuren

Nachfolgend werden Ausführungsformen anhand der beigefügten Figuren näher erläutert Es zeigen
- Figur 1: eine schematische Darstellung eines Endoskops mit einstellbarer Blickrichtungs;
- Figur 2: eine schematiche Darstellung des distalen Endes des Endoskops aus Figur 1;
- Figur 3: eine schematische Darstellung einer Ausführungsform des proximalen Endes des Endoskops aus den Figuren 1, und 2.
- Figure 4: eine weitere schematische Darstellung der Ausführungsformaus Figur. 3;
- Figur 5: eine schematische Darstellung einer weiteren Ausführungs des proximalen Endes des Endoskops aus den Figuren 1 und 2
- Figur 6: eine weitere schematische Darstellung der Ausführungsform aus Figur 5;
- Figur 7: eine schematische Darstellung einer weiteren Ausführungsform des proximalen Endes des Endoskops aus den Figuren 1 und 2;
- Figur 8: eine weitere schematische Darstellung der Ausführungsform aus Figur 7;
- Figur 9: ein schematisches Flussdiagramm

### Beschreibung von Ausführungsformen

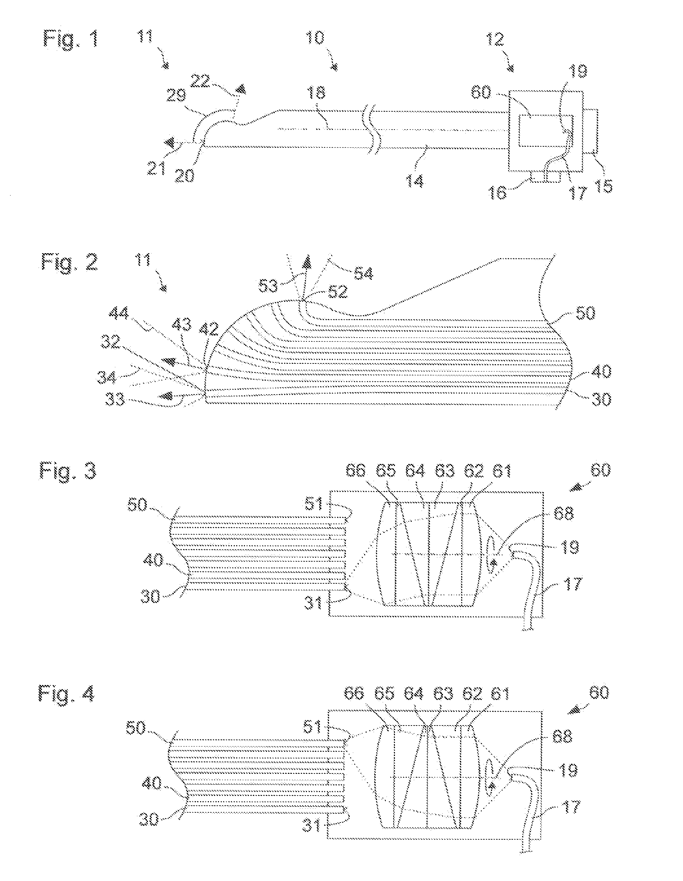

Figur 1 zeigt eine schematisch Darstellung eines Endoskops 10 mit einem distalen Ende 11, einem proximalen Ende 12 und einem starren schaft 14 der sich vom distalen Ende 11 bis zum proximalen Ende 11 erstreckt Alternativ ist der Schaft 14 flexibel oder teilweise flexibel. Der Querschnitt des Schafts 14 oder zumindest die äußere Kontur des Querschnitts des Schafts 14 ist zwischen dem distalen Ende 11und dem proximalen Ende 12 konstant oder im Wesentlichen konstant,Insbesondere ist die Kontur des Querschnitts des Schafts 14 kreinförming oder elliptisch. In diesem Fall ist die in Figur 1 dargestellt Längsachse 18 des Endoskops 10 die Symmetricachse der Mantelfläche des Schafts 14 zwischen dem distalen Ende 12 und dem proximalen Ende 11 Bei einer zylindrischen Mantelfläche des Schafts 14 ist die Längsachse 18 auch die Menge der Mittelpunkte oder Flächenschwerpunkte der Querschnitte des Schafts 14 zwischen dem distalen Ende 12 und dem proximalen Ende 1 1 ,Bei einer kreiszylindrischen Mantelfläche des schafts 14 ist die Längsachse 18 auch die Symmetrieachse der Mantelfläche

Am distalen Ende 12 weicht die Gestalt des Schafts 14 von der zylindersymmetrie ab, wie dies beispielhaft in Figur 1 dargestellt ist Insbesondere weist der Schaft 14.am distalen Ende 12 eine öffnung auf, die durch ein transparentes Fensterbauteil mit einer gewölbten Oberfläche 20 verschlossen ist Insbesondere verschließt das Fensterbauteil mit der gewölbten Oberfläche 20 die öffnung hermiefisch dicht. Die Oberfläche 20 des Festerbanteils hat beispielsweise die Gestalt eines Ausschnitts eines Kreiszylindermantels, wobei die Symmetrieachse des Kreiszylinders senkrecht zur Längsachse18 des Endoskops 10 und zur Zeichenebene der Figur 1 ist Alternative hat die Oberfläche 20 des transparenten Fensterbauteils die Gestalt eines Ausschnitts einer Kugeloberfläche oder eines rotationssymmetrischen oder nicht rotationssymmetrischen Ellipsoids.

Am distalen Enden 12 des Endoskops 10 sind im Schaft 4 optische Einrichtungen angeordnet, die in Figur 1 nicht dargestellt sind und eine Variation der Blickrichtung des Endoskops zwischen einer ersten extremen Blickrichtung 21 und einer zweiten extremen Blickrichtung 22 ermöglichen. Die Blickrichtung ist zwischen den beiden extremen Blickrichtungen 21, 22 insbesondere um eine Schwenkachse schwenkbar,die senkrecht zur Zeichenebene der Figur 1 ist Die Blickrichtung ist jeweils die Richtung bezogen auf das distale Ende 12 des Endoskops 10, in der ein Gegenstand liegt, der in der Mitte eines mittels des Endoskops 10 erfassten Bilds erscheint.

Bei dem in Figur 1 dargestellten Beispiel ist die erste extreme Blickrichtung 21 parallel oder im Wesentlichen parallel zur Längsachse 18 des Endoskops 10, Zwischen den extremen Blickrichtungen 21, 24 liegt ein Winkelbereich 29, der bei dem dargestellten Beispiel ca. 120 Grad umfasst. Innerhalb dieses Winkelbereichs ist die Blickrichtung des Endoskops 10 insbesondere kontimiierlich verstellbar.

Am proximalen Ende 11 weist das Endoskop 10 eine erste Kupplung 15 zum optischen Koppeln des Endoskops 10 mit einer Kamera oder ein Okular sowie eine zweite Kupplung 16 zum Koppeln des Endoskops 10 mit einer Lichtquelle über ein Lichtleitkabel auf_{.} Innerhalb des Schafts 14 ist eine Mehrzahl von Lichtwellenleitern angeordnet, die nachfolgend anhand der Figur 2 dargestellt werden. Ein kurzer proximaler Lichtwellenleiter 17 koppelt die zweite Kupplung 16 mit einer Lichtweicheneinrichtung 60, wobei eine Licht austrittsfläche 19 an der Lichtweicheneinchtung 60 angeordnet ist.

Figur 2 zeigt eine schematische vergröBerie Darstellung des distalen Endes 11 des oben anhand der Figur 1 dargestellten Endoskops 10. Dabei sind ähnlich wie in einer Schnittdarstellung mehrere Lichtwellenleiter 30, 40, 50 gezeigt, die im Inneren des Schafts 4 verlaufen. Um die Figur nicht zu überfrachten, sind nur drei der Lichtwellenleiter mit Bezugszeichen verschen.

Jeder der Lichtwellenleiter 30,40,50 endet in einer Lichtsustrittsflache 32, 42, 52 neben dem oben im Zusammenhang mit Figur 1 beschriebenen Fensterbauteil, das in Figur 2 nicht dargestellt ist Die Lichtaustrittsflächen 32, 42, 52 sind beispielsweise gebildet, indem die Enden der Lichtwellenleiter 30, 40, 50 mit Überstand und mittels eines Kitts in entsprechende Öffnungen des Gehäuses des Schafts 14 eingesetzt und anschlieβend his zur Kontur des Gehäuses des Schafts 14 zurtick geschliffen und poliert werden. Jeder Lichtwellenleiter 30, 40, 50 bestht insbesondere aus einem Bündel von Glas-oder Kunststofffasern. Die Lichtaustrittsflachen 32, 42, 52 hilden einfache Lichtaustrittseinrichtungen. fasern. Die Lichtaustrittsflächen 32, 42, 52 bilden einfache Lichtaustrittseinrichtungen. Alternativ können Linsen, Abdeckgläser und/oder andere Lichtaustrittseinrichtungen vorgesehen sein.

Mittlere Abstrahlrichtungen 33, 43, 53 der Lichtaustrittsflächen 32, 42, 52 sind insbesondere durch die Richtungen der 30, 40, 50 unmittelber an den Lichtaustrittsflächen 32, 42, 52 definiert. Die mittleren Abstrahlrichtungen 33, 43, 53 entsprechen insbesondere im Wesentlichen den Flächennormalen der Lichtaustrittsflächen 32, 42, 52,

Aus einer Lichtaustrittsfläche 32, 42 52 eines Lichtwellenleiters 30, 40, 50 austretendes Beleuchtungslicht breitel sich jedoch nicht nur in der mittleren Abstrahlrichtung, sondern innerhalb eines Lichtkegels 34, 44, 54 aus. Die Intensitat bzw. Strahlungsleistung nimmt ausgehend von der mittleren Abstrahlrichtung 33, 43, 53 nach auβen bzw mit zumehmendem Winkel zur mittleren Abstrahlrichtung 33, 43, 53 ab, Die in Figur 2 angedeuteten Lichtkegel 34, 44, 54 sind beispielsweise die Raumwinkelbereiche, innerhalb derer die Intensitat oder die Strahlangsleichtung des von einer Lichtaustrittsflache 32, 42, 52 ausgehenden Beleuchtungslichts mindestens die Hälfte des Maximalwerts beträgt Die Verteilung bzw, Winkelabhangigkeit der Strahlungsleistung des von einer Lichtaustrittsfläche 32, 41, 52, ausgehenden Beleuchtungslichts ist aufgrund der Übertragungseigenschaften der Lichtwellenleiter 30, 40, 50 insbesondere durch die Einkopplung an den in Figur 2 nicht dargestellten proximalen Enden der Lichtwellenleiter 30, 40, 50 bestimmt.

Bei dem in Figur 2 dargestellten Beispiel uberlappen die von verschiedenen Lichtaustrittsflächen 32, 42, 52 ausgehenden Lichtkegel 34, 44, 54. Es existieren also Raumbereiche, in denen Gegenstände von Beleuchtungslicht, das von zwei oder mehr verschiedenen Lichtaustrittsflächen 32, 43, 52 ausgeht, mit nicht nur unwesentlicher Strahlungsleistung beleuchten werden.

Neben oder zwischen den Lichtaustrittsflachen 32, 42, 52 der Lichtwellenleiter 30, 40, 50 sind weltere Lichtaustrittsflächen von weiteren Lichtwellenleitern dargestelllt, die in den Figuren 2 bis 8 nicht mit Bezungszeichen verschen sind. Die Anzahl der im Endoskop 10 vorgeschenen Lichtwellenleiter und ihre Anordnung kann von den in Figur 2 dargestellten abweichen.

Die Figuren 3 bis 8 zeigen schematische Darstellungen mehrerer verschiedener Ausführungsformen des proximalen Endes 12 des oben anhand der Figuren 1 und 2 dargestellten Endoskops Die Figuren 3 bis 8 zeigen jeweils in der Art von Schnittdarstellungen im Inneren des Endoskops angeordnete Einrichtungen.

Die Figuren 3 und 4 zeigen schematische Darstellungen einer Ausführungsform des oben anhand der Figuren 1 und 2 dargestellten Endoskops 10, bei der die Lichtweicheneinrich tung 60 eine Drehprismeneinrichtung ist. Die Drehprismeneinrichtung 60. umfasst eine Lichteintrittslinse 61, ein erstes Prisma 62, ein zweites Prisma 63, ein drittes Prisma 64, ein viertes Prisma 65 und eine Lichtaustrittslinse 66.

Die Lichtaustrittsfläche 19 des kurzen proximalen Lichtwellenleiters 17 ist am Brennpenkt der Lichteintrittslinse 61 angeordnet. Eine dem ersten Prisma 62 zugewandte Lichtaustrittsseite der Lichteintrittslinse 61 ist eben und parallel zu einer Lichteintrittsfläche des ersten Prismas 62 angeordnet.

Das erste Prisma 62 und das zweite Prisma 63 sind miteinander gefogt, insbesondere mittels eines transparenten Kitts miteinander verbunden. Eine der Lichteintrittslinse 61 zugewandte Lichteintrittsflache des ersten Prismas 62 und eine dem dritten Prisma 64 zugewandten Lichtaustrittsflache des zweiten Prismas 63 sind ehen und parallel zueinander. Das erste Prisma 62 und das zweite Prisma 63 sind gemeinsam um eine Achse 68 rotierbar.

Das dritte Prisma 64 und das vierte Prisma 65 sind miteinander gefügt, insbesondere mittels eines transparenten Kitts miteinander verbunden. Eine dem zweiten Prisma 63 zugewandte Lichteintrittsflache des dritten Prismas 64 und eine der Lichtaustrittslinse 66 zugewandte Lichtaustrittsfläche des vierten Prismas 65 sind eben und parallel zueinander. Das dritte Prisma 64 und das vierte Prisma 65 sind gemeinsam um die Achse 68 rotierbar. Eine dem vierten Prisma 65 zugewandte Lichteintrittsflache der Lichtaustrittslinse 66 ist chen. Die Lichteintrittsflächen 31, 51 51 der Lichtwellenleiter 30, 40, 50 sind in einer Fokalfläche, insbesondere in einer Fokalbene der Lichtaustrittslinse 66 der Drehprismeneinrichtung 60 angeornet.

Das erste Prisma 62 und das zweite Prisma 63 weisen transparente Materialien, insbesondere Gläser, mit unterschhiedlichen Brechungsindizes auf. Das dritte Prisma 64 und das vierte Prisma 65 weisen transparente Materialien, insbesondere Gläser, mit unterschiedlichen Brechungsindizes auf. Das erste Prisma 62 und das vierte Prisma 65 können das gleiche Material aufweisen, ebenso können das zweite Prisma 63 und das dritte Prisma 64 das gleiche Material aufweisen.

Das erste Prisma 62 und das zweite Prisma 63 einerseits sowie das dritte Prisma 64 und das vierte Prisma 65 andereseits sind unabhangig voneinander um 68 rotierbar. Alternativ sind das erste Prisma 62 und das zweite Prisma 63 einerseits sowie das dritte Prisma 64 und das vierte Prisma 65 andererseits beispielsweise mechanisch so miteinander gekoppelt, dass sie stets gegenläufig rotiert werden.

Die Lichtaustrittsflache 19 des kurzen prowimalen Lichtwellenteiters 17 ist am Brennpunkt der Lichteintrittslinse 61 angeordnet. Die Lichteintrittslinse 61 wirkt kollimierend, so dass von der Lichtaustrittsflache 19 des kurzen proximalen Lichtwellenleiters Lichtwellenleiters von der Lichtaustrittsflache 19 des kurzen proximalen Lichtwellenleiters 17 ausgehendes Beleuchtungslicht innerhalb der Prismen 62, 63, 64 65 sich parallel oder im Wesentlichen parallel ausbreitet.

Die Lichteintrittsflachen 31, 51 der Lichtwellenleiter 30, 40, 50 sind an der Fokalfläche der 66 angeordnet. Das von der Lichteintrittslinse 61 kollimierte Beleuchtungslicht wird von der Lichtaustrittslinse 66 auf eine oder wenige Lichteintrittsflachen 31, 51 der Lichtwellenleiter 30, 40, 50, gebündelt, insbesondere fokassiert.

Da die Grenzfläche zwischen dem ersten Prisma 62 und dem zweiten Prisma 63 sowie die Grenzfläche zwischen dem dritten Prisma 64 und dem vierten Prisma 65 nicht senkrecht zur Aubreitungsrichtung des Kollimierten Beleuchtungslichts sind, findet an diesen Genzflächen jeweils Brechung statt. Die Lichtausbreitung ändert sich durch die Brechung an den beiden Grenzflächen jeweils innerhalb einer Ebene, die durch die Licht-ausbreitungsrichtung und die Flächennormale der Grenzfläche definiert ist. Durch Rotation des ersten Prismas 62 und des zweiten Prismas 63 und/oder durch Rotation des dritten Prismas 64 und des vierten Prismas 65 um die Rotationsachse 68 werden die Flächennor-male der Grenzfläche zwischen dem ersten Prisma 62 und dem zweiten Prisma 63 und die Flächennormale der Grenzfläche zwischen dem dritten Prisma 64 und dem vierten Prisma 65 auf einem Kegelmantel um die Rotationsache 68 rotiert. Somit könne durch Rotation des ersten Prismas 62 and des zweiten Prismas 63 und/oder durch Rotation des dritten Prismas 64 und des vierten Prismas 65 um die Rotationsachse 68 die Richumgsanderungen an beiden Grenzflachen eingestellt werden.

Beispielhaft sind in den Figuren 3 und 4 zwei verschiedene Positionen des ersten Prismas 62 und des zweiten Prismas 63 and gleichzeitig zwei verschiedene Positionen des dritten Prismas 64 und des vierten Prismas 65 dargestellt. Bei den in Figur 3 dargestellten Positionnen des ersten Prismas 62 and des zweiten Prismas 63 bzw des dritten Prismas 64 and des vierten Prismas 65 wird von der Lichtaustrittsfläche 19 ausgehendes Beleuchtungsticht in die Lichteintrittsfläche 31 des ersten Lichtwellenleiters 30 eingekoppelt. Bei der in Figur 3 dargestellten Position der Prismen 62, 63, 64, 65 wird somit Beleuchtungalicht mittels des ersten Lichtwellenleiters 30 zum distalen Ende 11 des Endoskops 10 ubertragen. Dadurch wird der in Figur 2 gezeigte von der Lichtaustrittsfläsche 32 des ersten Lichtwettenleiters 30 ausgehende Lichtkegel 34 erzeugt

Bei den in Figur 4 dargestellten Positionen des ersten Prismas 62 und des zweiten Prismas 63 bzw des dritten Prismas 64 und des vierten Prismas 65 wird von der Lichtaustriusfläche 19 des kurzen proximalen Lichtwellenleiters 17 ausgehendes Beleuchtungslichf in die Lichteintrillsfläche 51 des dritten Lichtwellenleiters 50 eingekoppelt. Bei der in Figur 4 gezeigten Position der Prismen 62, 63, 64, 65 wird Beleuchtungslicht mittels des dritten Lichtwellenleitern 50 zundistalen Ende II des Endoskops 10 Ubertragen Dadurch wird der in Figur 2 gezeigte von der Lichtaustrittsflache 52 des dritten Lichtwellenleinters 50 ausgehende Lichtkegel 54 erzeugt.

Bel einer unabhängigen Rotation des ersten Prismas 62 und des zweiten Prismas 63 einerseits und des dritten Prismas 64 und des vierten Prismas 65 andererseits kann von der Lichtaustrittsfläche 19 des kurzénproximaten Lichtwellenleiters 17 qusgebendes Beleuchnungslicht auf in einem zweidimensionalen Array angeordnete Lichteintrittslfächen 31, 51 von Lichtwellenleitern 30,40, 50 gebündelt und in diese eingekoppelt werden.

Wenn das erste Prima 62 und das zweite Prisma 63 einerseits und das dritte Prisma 64 und das vierte Prisma 65 andererseits so miteinander gekoppelt sind, dass sie nur gegentäufig rotierbar sind, kann von der Lichtaustrittslfäche 19 des kurzen proxinten Lichtwellenleilers 17 ausgehendes Beleuchtungslicht auf Lichteintrittsflächen 31, 51 von Lichtwellenleitern 30, 40, 50, die entlang einer Linie, insbesonder auf einer Geraden, angeordacte sind, gebündelt und in diese eingekoppelt werde.

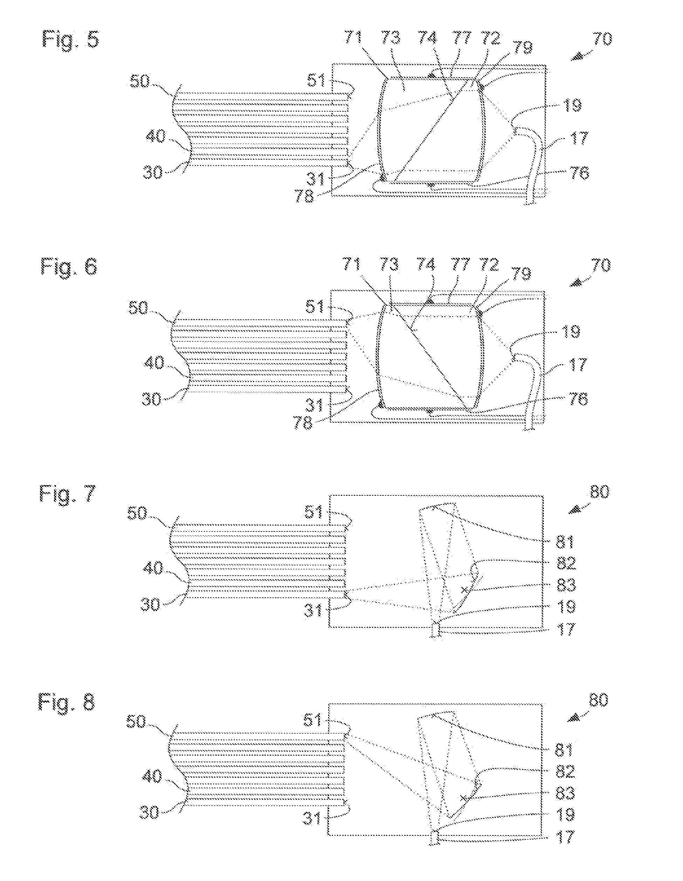

Die Figuren 5 und 6 zeigen schematische Darstellungen einer weiteren Ausführungsfrorm des obe, anhand der Figuren 1 und 2 dargestellten Endoskops 10. Bei dieser Ausfübrungsform ist anstelle der oben anhand der Figuren 3 und 4 dargestellten Drehprismeneinrichtung 60 eine Flüssiglinseneinrichtung 70 als Lichtweichneinrichtung vorgesehen. Die Flüssiglinseneinrichtung 70 umfast einen Hoshlkörper 71. is dem eine erste Flüssigkeit 72 und eine zweilte Flüssigkeit 73 angeordnet sind. Die erste Flüssigkeit 72 und die zweite Flüssigkeit 73 weisen unterschiedliche Brechungsindizes auf und sind nicht mischbar, Beispielsweise sind die erste Flüssigkeit 72 hydrophil bzw lipophob und die zweite Flüssigkeit 73 lipophil bzw. hydrophob. Zwischen der ersten Flüssigkeit 72 und der zweiten Flüssigkeit 73 hildet sich eine Grenzfläche 74 aus, die bein den in den Figuren 5 und 6 dargestellten Beispielen jeweiln ehen ist.

Am trasparenten Hohlkörper 51 sind eine erste Elektrode 76, eine zweite Elecktrode 77, eine dritte Elektrode 78 und eine vierte Elektrode 79 angeordnet. Weitere Elektronet. Weitere Elektroden, die in den Figuren 5 und 6 nicht daregestellt sind, können heispielsweise vor bzw, binter der Zeichenebene der Figuren 5 und 6 angeordnet sein. Zumindest die der Lichtaustrittsfläche 19 des kurzen proximaten Lichtwellenteiters 17 zugewandte Seite des Hohlkërpers 71 und die den Lichteintrittsflächen 31, 51 der Lichtwellenleiter 30, 40, 50 zugewandte Seite den Hohlkärpers 71 sowie die an diesen beinden Seiten des Hoblkörpers 71 angeordneten Elektroden 78, 79 sind transparent, urn eine Transmission von Beleuchtungslicht zuzulassen.

Die der Lichtaustrittsfläche 19 des kurzen proximalen Lichtwellenleiters 17 zugewandte Seite und die den Lichtteintrittsflächen 31, 35 der Lichtwellenleiter 30, 40, 50 zugewandte Seite des Hohlkörpers 71 sind, wie in den Figuren 5 und 6 angedentet, gewölbt, um Beleuchtungslicht zu kollimieren bzw zu fokussieren. Insbesendere sind die Lichtaustritts-flüche 19 des kurzen proximaten Lichtwellenleiters 17 und die der Lichtaustrittsfläche 19 zugewandte Lichteintrittsseite des Hohlkörpers 71 so angeardnet und die Lichteintrittsseite so gewölht, dass von der Lichtaustrittsfläche 19 des kurzen proximaten Lichtwellenleiters 17 ausgehendes Beleuchtungslicht innerhalb des Hohlkörpers 71 und der Flüssigkeiten 72, 73 sich parallel ausbreitet. Die Lichteintrittsflächen 31, 51 der Lichtwellenleiter 30, 40, 50 und die den Lichteintrittsflächen 31, 51 zugewandte Lichtaustrittsseite des Hohlkörpers 71 sind so angeordnet and Letztere so gewölbt, dass innerhalb des Hohlkörpers 71 und der Flüssigkeiten 72, 73 sich parallel ausbreitendes Beleuchtungslicht auf eine oder wenige Lichteintriusflächen 31, 51 gebündelt bzw, fokussiert wind.

Zumindest entweder die erste. Flüssigkeit 72 oder die zweite Flüssigkeit 73 ist so ausgehildet, dass sie mit éinem elektrischen Feld wechselwirkt Durch Antegen von elektrostatischen Spannungen an die Elektroden 75, 76, 77, 78 kann somit die Grenzfläche 74 geometrisch werändert werden. Insbsendere kann die Grenzfläche 74 in verschiedene gekippte Anordnungen bzw. Posisionen gebracht werden, die in den Figuren 5 und 6 angedeutet sind, und in denen die Grenzfläche 74 jeweils eben ist. Abweichend von den Darstellungen in den Figuren 5 und 6 kann die Grenzfläche 74 zwischen der ersten Flüssigkeit 72 und der zweiten Flüssigkeit 73 ferner gekrümmi werden. Durch eine Krümmung der Grenzfläche 74 zwischen der ersten Flüssigkeit 72 und der zweiten Flüssigkeit 73 kann die Bündelung oder Fekussierung des von der Lichtaustrittsfläche 19 des kurzen proximalen Lichtwellenleiters 17 usgehenden Beleuchtungalichts verändert werden, insbesondere kann die Anzahl der Lîchteintrîttsflächen 31, 51, n die das elenchtungslicht gleichzeitig eingekoppelt wind, werändert werden.

Beir der in Figur 5 dargestellten Anordnung der Grenzfläche 74 wind Belechungslicht minels des ersten Lichtwellenleiters 30 zum distanten Ende II des Endoskops 10 übertagen. Dadurch wird der in Figur 2 gezeigte von der Lîchtaustrittsfläche 32 des ersten Lichtwelleneleiters 30 ausgehende Lichtkegel 34 erzeugt.

Bei der in Figur 6 gezeigten Anordnung der Grenzfläche 74 wird Beleuchtungslicht mittels des dritten Lichtwellenleiters 50 zum distalen Ende II des Endoskops 10 übertragen. Dadurch wird der in Figur 2 gezeigte von der Lichtaustrittsfläche 52 des dritten Lichtwellenleiters 50 ausgehende Lichtkegl 54 erzeugt.

In weiteren Anordnungen der Grenzfläche 74 zwischen den in den Figuren 5 und 6 dargestellten Anordnungen wird von der Lichtaustrittsfläche 19 des kurzen proximalen Lichtwellenleiters 17 ausgehendes Beleuchtungslicht auf eine oder mehrere der Lichteintrittsflächen zwischen der Lichteintrittsfläche 31 des ersten Lichtwellenleiters 30 und der Lichteintrittsfläche 51 des dritten Lichtwellenleiters 50 gebündelt bzw, in diese eingekoppelt.

Die Figuren 7 und 8 zeigen schematische Darstellungen einer weiteren Ausflührungsform des proximalen Endes 12 des oben anhand der Figuren 1 und 2 dargestellten Endoskops 10, Bei dieser Ausführungsform ist anstelle der oben anhand der Figuren 3 und 4 dargestellten Drehprismeneinrichtung 60 oder der oben anhand der Figuren 5 und 6 dargestellten Flüssiglînseneinrichtung 70 eine Schwenkspiegeleinrichtung 80 als Lichtweicheneinrichtung vorgesche. Die Schweenkspiegeleinrichtung 80 umfasst eine erste reflectierende Fläche 81 und eine zweite reflektierende Fläche 82 Jede der beiden relektierenden Flächen 81, 82 ist beispielsweise eine reflektierende Fläche eines Spiegels oder eine verspiegelte oder totalreflektierende Fläche eines Prismas order eines anderen transparenten Körpers.

Zumîndest entweder die erste reflektierende Fläche 81 oder die zweite reflektierende Fläche 82 ist konvex gekrümmt, bei dern in den Figuren 7 und 8 dargestellten Beispielen sind beide reflektierenden Flächen 81, 82 konvex gekrümmt. Von der Lichtaustrittsfläche 19 des kurzen proximalen Lichtwellenleiters 17 ansgehendes Beleuchtungslicht wird nacheinander zunächst von der ersten reflektierenden Fläche 81 und dann von der zweiten reflektierenden Fläche 82 reflektiert und auf eine oder wenige Lichteintrittsflächen 31, 51 der Lichtwellenleiter 30, 40, 50 gebündelt bzw. fokussiert und in diese eingekoppelt. Insbesondere sind die erste reflektierende Fläche 81 und die zweite reflektierende Fläche 82 so ausgebildet und angeordnet dass von der Lichtaustrittsfläche 19 des kurzen proximalen Lichtwellenleiters 17 ausgehendes Beleuchtungslicht zwischen der ersten reflektierenden Fläche 81 und der zweiten reflektierenden Fläche 82 sich parallel ausbreitet. Die erste reflektierende Fläche 81 wirkt somit kollimierend. Dazu ist insbesondere die Lichtaustrittsfläche 19 des kurzen proximaten Lichtwellenleiters 17 an einem Fokus der ersten reflektierenden Fläche 81 angeordnet.

Zumindest entweder die erste reflektierende Fläche 81 order die zweite reflektierende Fläche 82 sind ferner um eine Achse schwenkbar. Bei dem in den Figuren 7 und 8 dargestellten Beispiel ist die zweite reflektierende Fläche 82 um eine Achse 83 schwenkbar, die im Wesentlichen senkrecht zur Zeichenebene der Figur 7 ist. Durch Schwenken der zweiten reflektierenden Fläche 82 um die Schwenkachse 83 ist einstellbar, auf welche der Lichteintrittsflächen 31, 51 der Lichtwellenteiter 30, 40, 50 Beleuchtungalicht gebündeh bzw fokussier wird, das von der Lichtaustrittsfläche 19 des kurzen proximaten Lichtwellenleiters 17 ausgeht.

Bei der in Figur 7 dargestellten Position der zweiten reflektierenden Fläche 82 wird von der Lichtaustrittsfläche 19 des kurzen proximaten Lichtwellenleiters 17 ausgehendes Beleuchtungslicht von den reflektierenden Flächen 81, 82 auf die Lichteintrittsfläche 31 des ersten Lichtwellenleiters 30 gebündelt bzw in diese eingekoppelt. Bei der in Figur 7 dargestellten Position der zweiten reflektierenden Fläche 82 wird somit Beleuchtangslicht mittels des ersten Lichtwellenleiters 30 zum distalen Ende II des Endoskops 10 äbertragen. Dadurch wird der in Figur 2 gezeigte von der Lichtsustrittsflache 32 des ersten Lichtwellenleiters 30 ausgehende Lichtkegel 34 erzeugt.

Bei der in Figur 8 dargestellten Position der zweiter reflektierenden Fläche 82 wird von der Lichtanstritisfläche 19 des kurzen proximalen Lichtwellenleiters 17 ausgëhendes Beleuchtungslicht von den reflektierenden Flächen 81, 82 auf die Lichteintrittsfläche 51 des dritten Lichtwellenleiters 50 geändelt bzw, in diese eingekoppelt. Bei der in Figur 8 dargestellten Position der zweiten reflektierenden Fläche 82 wird somit Beleuchtungsficht mittels des dritten Lichtwellenleiters 50 zum distalen Ende 11 des Endoskops 10 übertragen, Dadurch wird der in Figur 2 gezeigte von der Lichtaustrittsfläche 52 des dritten Lichtwellenleiters 50 ausgehende Lichtkegel 54 erzeugt.

In weiteren Pisitionen der zweiten reflektierenden Fläche 82 zwischen den in den Figuren 7 and 8 dargestellten Positionen wird von der Lichtausträtsfläche 19 des kurzen proximalen. Lichtwellenleiters 17 ausgehendes Beleachtungslicht auf eine oder mehrere der Lichteintrittsflächen zwischen der Lichteintrittsfläche 31 des ersten Lichtwellenleiters 30 and der Lichteintrittsfläche 51 des dritten Lichtwellenleiters 50 gebündelt bzw in diese eingekoppelt.

ln Figur 2 sind die Lichtaustrittsflächen 32. 42. 52 der Lichtwellenleiter 30, 40, 50 beispielhaft so angeordnet, dass zwischen den mittleren Abstrahlrichtungen 33, 43, 53 benachbarter Lichtaustrittsflächen 32, 42, 52 jeweils gleiche oder im Wesentlichen gleich Winkel liegen. Lichtaustrittsflächen 32, 42, 52 können entlang zweier gegenäberliegender Ränder des Fensterbauteils des Beobachtungsstrahlengangs angeordnet sein. In diesen Fall können die Lichtaustritisflächen 32, 42, 52 so angeordnet sein, dass die mittleren Abstrahlrichtangen 33, 43, 53, der Lichtaustrittflächen gegeneinander versetzt sind. Beispielsweise bilden die mittleren Abstrahlrichtungen 33, 43, 53 der Lichtaustrittsflächen an einer Seite des Fensterbauteils zur Längsachse 18 des Endoskops Winkel von―10 Grad, +10 Grad, +30 Grad, +50 Grad, +70 Grad, 490 Grad, und die mittleren Abstrahlrichtungen 33, 43, 53 der Lichtaustrittsflächen an der gegenüberliegenden Seite des Fensterbauteils bilden zur Längsachse 18 des Endoskops Winkel vib 0 Grad, +20 Grad, +40 Grad, +60 Grad, +80 Grad, +100 Grad und +120 Grad.

lasbesendere bei dem zuletzt genannten Beispiel der Anordnung der Lichtaustrittsfläche 32, 42, 52, aber auch bei anderen Anordnungen der Lchtaustrittsfläche 32, 42, 52, können die Lichteintrittsflächen 31, 51 der Lichtwellenleiter 30, 40, 50 anders als in den Figuren 3 bis 8 dargestellt angeordner sein. ln den Figuren 3 bis 8 sind die Lichteintrittsflächen 31, 41 51 beispielhaft in gleichen gegenseitigen Abständen angeordnet. Abweichend davon kann es je nach Anordnung der Lichtaustrittsflächen 32, 42, 52 am distalen Ende 11 des Endoskops 10 vorteilhaft sein, die Lichteintrittsflächen 31, 41, 51 anders anzuordnen, beispielsweise paarweise gruppiert.

Ferner können abweichend von den Darstellungen in den Figuren 3 bis 8 Kegel bzw. Tapeer, insbesondere Faserkegel oder Vollkegel, an den Lichteintrittsflächen 31, 41, 51 der Lichtwellenleiter 30, 40, 50 vorgeschen sein. Mittels eines derartigen Kegels kann die Winkelverteilung des in, einen Lichtwellenleiter eingekoppelten Belenchtungslichts verändert bzw, eingestellt werden. Aufgrand der Übertragungseigenschaften der Lichtwellenleiter 30, 40, 50 können so die Öffnungswinkel der von den Lichtaustrittsflächen 32, 42, 52 der 38, 40, 50 ausgehenden Lichtkegel 34, 44, 45 eingestellt werden.

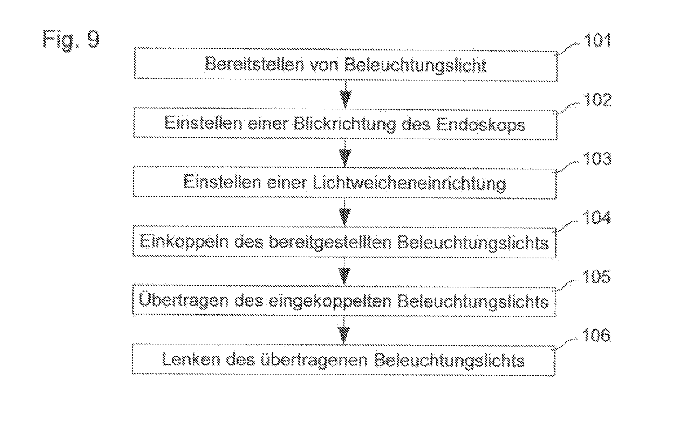

Figur 9 zeigt ein schematisches Flassdiagramm eines Verfahrens zum Beleuchten eines Sichtfelds in einer einstellbaren Blickrichtung eines Endoskops. Obwohl dieses Verfahren auch mittels eines Endoskops ausführbar ist, das sich von den oben anhand der Figuren 1 bis 8 dargestellten Endoskopen unterscheidet, werden nachfolgend zum besseren Verständnis Bezugszeichen aus den Figuren 1 bis 8 beispielhaft verwendet

Bei einem ersten Schritt 101 wird Beleuchtungslicht bereitgestellt. Das Beleuchtungslicht wird beispielsweise von einer Lichtquelle bereitgestellt, die mit dem Endoskop 10 über ein Lichtleitkahel und eine Kapplung 16 des Endoskops 10 gekoppelt ist. Alternativ wird das Beleuchtungslicht von einer in das Endoskop 10 integrierten Lichtquelle erzengt.

Bei einem zweiten Schritt 102 wird eine Blickrichtung 21, 22 das Endoskops 10 eingestellt. Dazu wird beispielsweise ein Prisma oder ein Spiegel am distalen Ende 11 des Endoskops 10 geschwenkt. Bei einem dritten schritt 103 wird eine Lichtweicheneinrichtung 60; 70; 80 entsprechend der eingestellten Blickrichtung 21, 22 des Endoskops 10 eingestellt. Bei einem vierten Schritt 104 wird das bereitgestellte Beleuchtungslicht mittels der beim dritten Schrit 103 eingestellten Lichtweicheneinrichtung 60, 70, 80 in einen oder mehrere einer momentan eingestellten Blickrichtung 21, 22 zugeordnete Lichtwellenleiter 30, 40, 50 aus einer Mehrzahl von Lichtwellenleitern 30, 40, 50 eingekoppelt

Bei einem fünften Schritt 105 wird das bein vierten Schritt 104 eingekoppelie Belenchturgslicht mittels des oder der Lichtwellenleiter, in die das Beleuchtangslicht beim vierten Schritt 104 eingekoppelt warde, zu einer oder mchreren zugeordeneten Lichtaustrittseinrichtungen 32, 42, 52 am distalen Ende 11 des Endoskops 10 übertragen. Bei einem sechsten Schritt 106 wird das beim fünften Schritt 105 mittels eines Lichtwellenleiters übertragene Belenchtungslicht mittels einer dem Lichtwellenleiter zugeordneten Lichtaustrittseinrichtungen 32, 42, 52 in einen dem Sichtfeld entsprechenden Raumwinkelbereich 34, 44, 54 gelenkt.

### Bezugszeichen

- 10: Endoskop
- 11: distales Ende des Endoskops 10
- 12: proximales Ende des Endoskops 10
- 14: Schaft des Endoskops 10
- 15: erste Kapplung
- 16: zweite Kupplung
- 17: kurzer proximaler Lichtwellenleiter
- 18: Längsachsedes Endoskops 10
- 19: Lichtaustrittsfläche des kurzen proximalen Lichtwellenleiters 17
- 20: Oberfläche eines Fensterbauteils
- 21: erste extreme Blickrichtung (0°)
- 22: zweite extreme Blickrichtung (120)
- 28: Schwenkachse der Blickrichtung 21, 22
- 29: Winkelbereich der Blickrichtungen
- 30: erster Lichtwellenleiter
- 31: Lichteintrittfläche des ersten Lichtwellenleiters 30
- 32: Lichtaustrittsfläche des ersten Lichtwellenleiters 30
- 33: mittlere Abstrahlrichtung der Lichtaustrittsfläche 32 des ersten Lichtwellenleiters 30
- 34: von der Lichtaustrittsfläche 32 des ersten Lichtwellenleiters 30 ausgehender Lichtkegel.
- 40: zweiter Lichtwellenleiter
- 41: Lichteintrittfläche des zweiten Lichtwellenleiters 40
- 42: Lichtaustrittsfläche des zweiten Lichtwellenleiters 40
- 43: mittlere Abstrahlrichtung der Lichtaustrittsfläche 42 des zweiten Lichtwellenleiters 40
- 44: von der Lichtaustrittsfläche 42 des zweiten Lichtwellenleiters 40 ausgehender Lichtkegel
- 50: dritter Lichtwellenleiter
- 51: Lichteintrittfläche des dritten Lichtwellenleiters 50
- 52: Lichtaustrittsfläche des dritten Lichtwellenleiters 50
- 53: mittlere Abstrahlrichtung der Lichtaustrittsfläche 52 des dritten Lichtwellenleiters 50
- 54: von der Lichtaustrittsfläche 52 des dritten Lichtwellenleiters 50 ausgehender Lichtkegel

- 60: Drehprismneinrichtung
- 61: Lichteintrittslinse er Drehprismeneinrichtung 60
- 62: erstes Prisma der Drehprismeneinrichtung 60
- 63: zweites Prisma der Drehprismeneinrichtung 60
- 64: drittes Prisma der Drehprismeneinrichtung 60
- 65: viertes Prisma der Drehprismeneinrichtung 60
- 66: Lichtaustrittslinse der Drehprismeneinrichtung 60
- 68: Rotationsachse der Prismen 62, 63, 64, 65 der Drehprismeneinrichtung 60

- 70: Flussiglinseneinrichtung
- 71: transparenter Hohlkörper der Flässiglinseneinrichtung 70
- 72: erste Flässigkeit
- 73: zweite Flässigkeit
- 74: Grenzfläche zwischen der ersten Flässigkeit 72 und der zweiten Flüssigkeit 73
- 76: erste Elektrade am transparenten Hohlkörper 71
- 77: zweite Elektrode am transparenten Hahikörper 71
- 78: dritte Elektrode am transparenten Hohlkörper 71
- 79: vierte Elektrode am transparenten Hohlkörper 71
- 80: Schwenkspiegeleinrichtung
- 81: erste reflektierende Fläche
- 82: zweite reflektierende Fläche
- 83: Schwenkachse der zweiten reflektierenden Fläche 82
- 101: erster Schritt
- 102: zweiter Schritt
- 103: dritter Schritt
- 104: vierter Schritt
- 105: fünfter Schritt
- 106: sechster Schritt

## Patentansprüche

1. Endoskop (10) mit einstellbarer Blickrichtung (21, 22), mit:
einer Mehrzahl von Lichtaustriltseinrichtungen (32, 42, 52) am distalen Ende (11) des Endoskops (10), wobei die Lichtanstrittseinrichtungen (32, 42, 52) ausgebildet und angeordnet sind, um aus verschiedenen Lichtaustrittseinrichtungen (32, 42, 52) austretendes Beleuchtungslicht in zumindest teilweise unterschiedliche Raumwinkelbereiche (34, 44, 54) zu leiten;
mehreren Lichtwellenleiters (30, 40, 50) zum. Leiten von Beleuchtungslicht zu den Lichtaustrittseinrichtungen (32, 42, 52);
einer Lichtweincheneinrichtung (60; 70; 80) zum steuerbaren Einkoppeln von Beeuchtungslicht in einen oder mehrere der Mehrzahl von Lichtwellenleiter (36).

2. Endoskop (10) Nach dem vorangehenden Ansprach, bei dem die Lichtweicheneinrichtung (60) ein in mehrere Positionen beweghares Prisma (62, 63, 64, 65) umfasst, wobei bei verschiedenen Positionen des Prismas (62, 63, 64, 65) Beleuchtungslicht in verschiedene Lichtwellenleiter (30, 40, 50) einkoppelbar ist.

3. Endoskop (10) nach einem der vorangehenden Ansprüche, bei dem die Lichtweicheneinrichtung (60) ein rotierbares Paar miteinander starr verbandener Prismen (62, 63, 64, 65) umfasst.

4. Endoskop (10) nach einem der vorangehenden Ansprüche, bei dem die Lichtweicheneinrichtung (60) zwei relativ zueinander rotierbare Paare von Prismen (62, 63, 64, 65) umfasst.

5. Endoskop (10) nach Anspruch 1, bei dem Lichtweicheneinrichtung (70) zwei nicht mischbare Flässigkeiten (72, 73 ) mit unterschiedlichen Brechungsindizes und eine Grenzflâchensteuereinrichtung (76, 77, 78, 79) zum elektrischen oder magnetischen Verändern zamadest emweder der Anordoung oder der Form einer Grenzfläche (74) zwischen den nicht mischbaren Fluseigkeiten (72, 73) amfasst

6. Endoskop (10) nach dem vorangehenden Anspruch, bel dem die Grenzflächensteuereinrichung mehrere Eledtroden (76, 77, 78, 79) umfasst, wobei die Flüsslgkeiten (72, 75) so ausgebildet und die Elektroden (76, 77, 78, 79) so ausgebildet und angeordnet sind, dass durch Anlegen einer elektrostatischen Spannung an die Elektroden (76, 77, 78, 79) die Grenzfläche (74) zwischen den nicht mischbaren Flüssigkeiten (72, 73) zumindest entweder verschoben oder verformt werden kann

7. Endoskop (10) nach Anspruch 1, bei dern die Lichtwencheneinrichrung (80) eine in mehrere Positionen heweghare reflektierende Fläche (81, 82) zum Umlenken von Beleuchtungslicht amfasst, wobei bei verschiedenen Positionen der reflektierenden Fläche (81, 82) Beleuchtumgsficht in verschiedene Lichtwellenleiter (30, 40, 50) einkoppelbar ist.

8. Endoskop (10) nach einem der vorangehenden Ansprüche, bei dem Lichteinriftsflächen (35) der Lichtwellenleiter (36) und die Lichtweicheneinrichtung (40, 50, 60) am proximalen Ende (12) des Endoskops (10) angeordnet sind.

9. Verfalmen zum Beleuchten eines Sichtfelds in einer einstellbaren Blichrichtung (21 , 22) eines Endoskops (10), mit folgenden Schriften:
Bereitstellen (101) von Beleuchtungslicht;
Einkoppeln (104) des bereitgestellten Belenchtungslicht in einen oder mehrere einer momentan eingestellten Blickrichtung zugeordnete Lichtwellenleiter (30, 40, 50) aus einer Mehrzahl von Lichtwellenleitern (30, 40, 50)
Übertragen (105) des eingekoppelten Beleuchtumgslichts mittels des oder der ausgewählten Lichtwellenleiter (30, 40, 50) zu einer oder mehreren zugenrdneten Lichtaustrittseinrichtungen (32, 42, 52) am distalen Ende (11) des Endoskops (10);
Lenken (106) des mittels eines Lichtwellenleiters (30, 40, 50) äbertragenen Beleuchtungslichts in einen dem Sichtfeld entsprechenden [def] Raumwinkelbereich (34, 44, 54.) mitteln einer dem Lichtwellenleiter zugeordneter Lichtaustrittseinrichtung (32, 42, 52).

10. Varfahren nach dem vorangehenden Anspruch, ferner mit folgenden Schritten
Einstellen (102) einer ausgewählten (21,22) des Endoskops (10);
Einstellen (103) einer Lichtweicheneinrichtung (60, 70, 80) entsprechend der eingestellten Blickrichtung (21, 22) des Endoskops (10).
